(19) Europäisches Patentamt
European Patent Office
Office européen des brevets

(11) EP 1 358 865 B2

(12) NEW EUROPEAN PATENT SPECIFICATION
After opposition procedure

(45) Date of publication and mention of the opposition decision:
**26.07.2017 Bulletin 2017/30**

(45) Mention of the grant of the patent:
**01.12.2010 Bulletin 2010/48**

(21) Application number: **03252202.1**

(22) Date of filing: **08.04.2003**

(51) Int Cl.:
*A61Q 5/12* (2006.01) *A61Q 5/10* (2006.01)
*A61K 8/898* (2006.01)

(54) **Durable hair treatment composition**

Haarpflegezusammensetzung mit langanhaltender Wirkung

Composition pour traitement capillaire durable

(84) Designated Contracting States:
**AT BE BG CH CY CZ DE DK EE ES FI FR GB GR HU IE IT LI LU MC NL PT RO SE SI SK TR**

(30) Priority: **22.04.2002 GB 0209136**

(43) Date of publication of application:
**05.11.2003 Bulletin 2003/45**

(73) Proprietor: **THE PROCTER & GAMBLE COMPANY
Cincinnati, OH 45202 (US)**

(72) Inventors:
- **Glenn, Robert Wayne
Virginia Water,
Surrey GU25 4HZ (GB)**
- **Godfrey, Simon Paul
Uxbridge,
Middlesey UB8 1AR (GB)**
- **McMeekin, Anthony
Bagshot,
Surrey GU19 5QY (GB)**
- **Boumard, Coralie Claude Monique
Egham,
Surrey TW20 8HG (GB)**
- **Bureiko, Andrei Sergeevich
Ascot,
Berskhire SL5 0BB (GB)**
- **Raineau, Olivier Charles
Richmond,
Surrey TW9 2PN (GB)**

(74) Representative: **Herzog, Fiesser & Partner
Patentanwälte PartG mbB et al
Isartorplatz 1
80331 München (DE)**

(56) References cited:

| | |
|---|---|
| EP-A- 0 275 707 | EP-A- 0 492 657 |
| EP-A- 0 574 156 | WO-A-01/25380 |
| WO-A-01/90477 | WO-A-02/47632 |
| WO-A1-00/45787 | GB-A- 2 141 454 |
| US-A- 4 450 152 | US-A- 4 620 878 |
| US-A- 5 942 216 | US-A- 5 981 681 |
| US-A- 6 153 570 | US-A1- 2001 008 917 |

- **ANONYMOUS: "Wacker-Belsil ADM 652, ADM 656, ADM 1100, ADM 1600, ADM 1650" INTERNET, [Online] January 2000 (2000-01), XP002258243 Retrieved from the Internet: <URL:http://www.wacker.com/internet/webcache/de_DE/PTM/TM/Wacker_Belsil/ADM/Wacker-B elsil_ADM_div_E.pdf?ts=1066372115918> [retrieved on 2003-10-17]**
- **ANONYMOUS: "Dow Corning 8803 Softener" INTERNET, [Online] 4 January 2001 (2001-01-04), XP002258244 Retrieved from the Internet: <URL:http://www.dowcorning.com> [retrieved on 2003-10-17]**
- **ANONYMOUS: "Dow Corning 2-8040 Polymer" INTERNET, [Online] 1 October 1999 (1999-10-01), XP002258821 Retrieved from the Internet: <URL:http://www.dowcorning.com> [retrieved on 2003-10-22]**
- **ANONYMOUS: "Dow Corning 8813 Polymer" INTERNET, [Online] 16 August 1999 (1999-08-16), XP002258822 Retrieved from the Internet: <URL:http://www.dowcorning.com> [retrieved on 2003-10-22]**
- **ANONYMOUS: "Dow Corning 2-8822A Polymer Silicone Softener" INTERNET, [Online] 1 October 1999 (1999-10-01), XP002258823 Retrieved from the Internet: <URL:http://www.dowcorning.com> [retrieved on 2003-10-22]**

EP 1 358 865 B2

- **DATABASE WPI Derwent Publications Ltd., London, GB; AN 1995-128213 XP002258245 & JP 07 053330 A (KAO CORPORATION), 28 February 1995 (1995-02-28)**
- **PATENT ABSTRACTS OF JAPAN vol. 012, no. 270 (C-515), 27 July 1988 (1988-07-27) & JP 63 051315 A (KAO CORP;OTHERS: 01), 4 March 1988 (1988-03-04)**
- **DATABASE WPI Derwent Publications Ltd., London, GB; AN 1995-128214 XP002258246 & JP 07 053331 A (KAO CORPORATION), 28 February 1995 (1995-02-28)**

## Description

### FIELD OF THE INVENTION

The present invention relates to topical compositions for treating hair. The compositions comprise functionalized silicones having defined physicochemical properties that exhibit superior conditioning duability on hair than previously known silicone based conditioners, especially where the hair has been previously damaged through chemical treatments, such as occurs during permanent dyeing, bleaching and permanent waving.

### BACKGROUND OF THE INVENTION

Oxidative dyeing, otherwise known as permanent colouring leads to irreversible physico-chemical changes to the hair. Typically, during this process, two components are mixed together prior to application to the hair. These components usually comprise an oxidising agent, such as hydrogen peroxide, and a dyeing material, such as oxidative dye precursors and couplers (buffered at a high pH, typically around 10). After contacting with the hair, the mixture is left for a period of time suitable to allow the required colour transformation to occur, after which the hair becomes more hydrophilic versus non-coloured hair due to irreversible chemical changes. While not wishing to be bound by theory, this change in hair hydrophilicity appears to be due, among other things, to the oxidation of the keratin-keratin cysteine amino acids within the hair creating more hydrophilic cysteic acid amino acid residues and the hydrolysis of the hair's natural hydrophobic, protective layer denoted as the F-Layer, a covalently attached lipid to the outer epicuticular envelope, 18-methyleicosanoic acid. This colouring process is usually repeated regularly by consumers in order to maintain their desired hair colour and colour intensity and also to ensure that new hair growth has the same colour as the older hair. As a consequence the hair changes polarity from a relatively hydrophobic surface near the scalp where it could be experiencing its first colour, to a progressively more polar substrate at the hair tips, which may have been subjected to multiple colouring treatments. A discussion of oxidation dyeing of hair can be found in "The Science of Hair Care" by Charles Zviak, Marcel Dekker, New York, 1986. These irreversible physicochemical changes can manifest themselves as increased roughness, brittleness and dryness leading to less manageable hair.

After the colouring process human hair becomes soiled due to its contact with the surrounding environment and from the sebum secreted by the scalp. This soiling of the hair causes it to have a dirty feel and unattractive appearance and necessitates shampooing with frequent regularity. Shampooing cleans the hair by removing excess soil and sebum, but can leave the hair in a wet, tangled, and generally unmanageable state. Once the hair dries, it is often left in a dry, rough, lustreless, or frizzy condition due to the removal of the hair's natural oils and other natural or deposited conditioning and moisturizing components. Hair can also be left with increased levels of static upon drying which can interfere with combing and result in a condition commonly referred to as "fly-away-hair". These conditions tend to be exaggerated on hair which has been previously oxidatively coloured.

It is known to use hair conditioners to alleviate the above problems. More specifically, it is known to add conditioning materials to colorant products or to supply them separately as part of colorant kits. It is also known to use conditioners in the shampooing process. These approaches range from post-shampoo application of hair conditioners such as leave-on or rinse-off products, to hair conditioning shampoos which attempt to both cleanse and condition the hair from a single product. Hair conditioners are typically applied in a separate step following shampooing. The hair conditioners are either rinsed-off or left-on, depending upon the type of product used.

Polydimethylsiloxanes (PDMS) are often employed as conditioning materials to improve hair feel. However, it is known that, in the case of more hydrophilic hair obtained after oxidative coloring, PDMS deposition is greatly reduced, and cannot provide the same benefit in hair condition as for non-oxidatively coloured hair.

Attempts appear to have been made in the prior art to solve the problems discussed above. To be more specific, there has been a move away from the use of highly hydrophobic PDMS-based silicones and towards using functionalized silicones, comprising functional groups such as amines - see, for example, EP 0 275 707 and WO 99/49836. The compositions disclosed by those documents are not durable, however, and are liable to be removed from the hair during the course of a couple of subsequent shampooings. This is especially the case for hydrophilic, oxidatively damaged hair. Creating a conditioner that does not need to be applied every time the hair is washed would be highly advantageous.

To obtain an improved conditioning effect, it is also important to ensure that enough silicone fluid deposits on each filament to meet consumer needs, i.e. that the absolute deposition of silicone fluid is sufficient for this purpose, both initially and long-term after subsequent shampooings.

With the above discussion in mind, the invention will ideally provide a hair treatment composition comprising a conditioning agent that deposits enough conditioning agent onto the hair to meet consumer needs, both in the case of virgin and multiple oxidation dyed hair, and which is durable, i.e. does not wash off so rapidly that the conditioning benefit is lost to the consumer.

## SUMMARY OF THE INVENTION

According to the invention, a hair treatment composition as claimed in claim 1 is provided, comprising a functionalized silicone polymer having an interfacial tension (IFT) of 1 to 12 mN/m and a viscosity from 400 to 150,000 mPa.s, wherein the functionalized silicone polymer deposits durably on hair.

As used herein, the term "functionalized" silicone includes polydimethylsiloxanes (PDMS) in which at least one methyl group has been replaced by a different group, which is preferably not hydrogen. The term "functional silicone" is synonymous with the term "functionalized silicone".

As used herein, the term "durable" used in relation to functionalised silicone deposition means that the Durability Index, as measured by the Silicone Durablity Index Method protocol, hereinbelow, is at least 0.20, preferably greater than 0.50, more preferably greater than 0.75, and most preferably greater than 1.0. Phrases such as "deposits durably" and durable deposition are to be interpreted accordingly.

The term HLB value is known to the skilled person working in this technical area - see for example Römpp Chemie Lexikon, Thieme Verlag, Stuttgart 9th, Edition, 1995 under "HLB-Wert".

## DETAILED DESCRIPTION

All cited references are incorporated herein by reference in their entireties.

All percentages given herein are by weight of total composition unless specifically stated otherwise. All ratios given herein are weight ratios unless specifically stated otherwise.

All molecular weights given herein are weight average molecular weights, unless stated otherwise.

In examining how to solve the above technical problems, the present inventors moved away from focusing exclusively on molecular properties and started also to consider what effect altering physical properties of silicones might have. That is because we observed that silicone droplets tend to interact with strands of hair predominantly as fluids and not as individual molecules. A number of parameters were investigated and matched against the objectives. We identified that, within a certain hydrophilicity range, advantageous technical benefits can be achieved as regards the absolute deposition and the durability of silicone deposition on hair. Hydrophilicity is traditionally measured by means of interfacial tension (IFT) which is conventionally established using a pendant drop-type method, as defined hereinbelow. The present inventors also used such a method. The hydrophilicity range according to the invention corresponds to an IFT of 1 to 12 mN/m, preferably 1 to 10 mN/m, more preferably 1 to 8 mN/m, most preferably from 1 to 4 mN/m.

The present inventors have also established that, for a given functional silicone hydrophilicity level, the silicone fluid viscosity has a profound influence on the level of durability and the tactile sensorial feel of the deposited silicones. Advantageously, the silicone has a viscosity in the range 400 - 150,000 mPa.s. More advantageously, the viscosity is in the range 600 - 100,000 mPa.s. More advantageously still, the viscosity is in the range 4000 - 25,000 mPa.s.

The inventors have established that functional silicone durability is highly dependant on a defined minimum viscosity. While not wishing to be bound by theory, it is believed that a functional silicone's durability is determined by its ability to self-emulsify water during the application rinse process by the consumer to create a structured deposit on hair with viscoelastic and thereby adhesive properties. In this process, a minimum viscosity is required to enable the water-in-silicone structure formation to progress irreversibly beyond a yield point creating the durable gel, less the water merely phase-separates from the silicone post removal of the energy from rinsing. The minimum silicone fluid viscosity for irreversible structure formation and hence durability has been determined to be approximately 400 mPa.s.

It has also been established by the inventors that the tactile feel or sensorial performance of the deposited functional silicones within the above defined requisite silicone hydrophilicity range and above the minimum viscosity for durability is improved dramatically above a silicone viscosity of 4,000mPa.s. While not wishing to be bound by theory, it is believed that the functional silicones with viscosity greater than 4,000mPa.s result in a more smooth and uniform structured water-in-silicone deposit morphology on hair vs. the structured deposits from fluids below 4,000mPa.s. This is believed to be due to the slower kinetics of structure formation during the application rinse process by the consumer (owing to the increased base fluid viscosity) enabling the fluid to still flow/spread more completely prior to the structure formation progressing beyond a yield point.

Suprisingly, the present inventors have determined that the benefits associated with functionalized silicones having a hydrophilicity and viscosity in the defined ranges apply regardless of chemistry, i.e. regardless of the functional groups concerned.

Hair treatment compositions according to the invention may comprise from 0.1 to 20wt%, preferably from 0.25 to 15wt%, more preferably from 0.5 to 10wt% and more preferably still from 0.5 to 7.5wt% functionalized silicone.

Functionalized silicones which may be incorporated into compositions according to the invention include organomodified silicones of the pendant or graft type wherein polar functional substituents are incorporated within or onto monovalent organic groups, $A^1$, $A^2$, $A^3$ and $A^4$ used hereinafter, as follows:

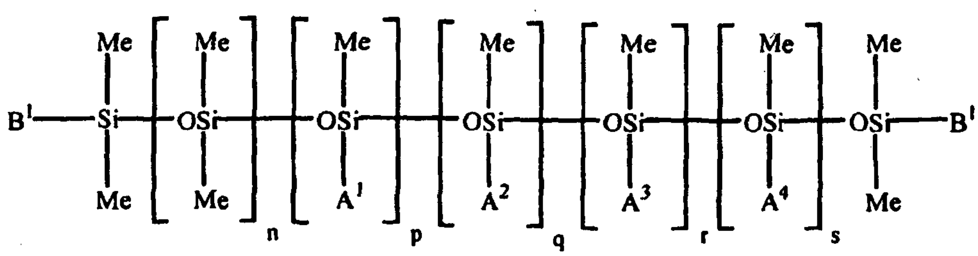

Also included are the organomodified silicones of the block copolymer type wherein these polar functional substituents are incorporated within or onto bivalent organic groups, $A^1$, $A^2$, $A^3$ and $A^4$ used hereinafter.

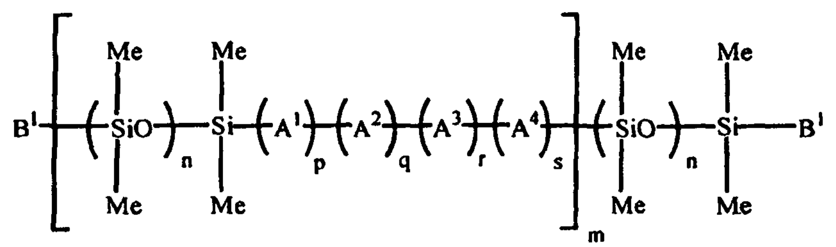

where m is greater than or equal to 1, n is about 50 to 2000, p is about 0 to 50, q is about 0 to 50, r is about 0 to 50, s is about 0 to 50, wherein p + q + r + s is greater than or equal to 1, $B^1$ is H, OH, an alkyl or an alkoxy group.

The above functionalized silicones of the pendant or block copolymer type can also incorporate silicone branching groups including $MeSiO_{3/2}$, known as silsesquioxane or T groups, and $SiO_{4/2}$, known as Q groups by those skilled in the art.

Organic groups $A^1$, $A^2$, $A^3$ and $A^4$ may be straight, branched or mono- or polycyclic aliphatic, mono or polyunsaturated alkyl, aryl, heteroalkyl, heteroaliphatic or heteroolefinic moiety comprising 3 to 150 carbon atoms together with 0-50 heteroatoms, especially O, N, S, P and can incorporate one or more polar substituents selected from electron withdrawing, electron neutral, or electron donating groups with Hammett sigma para values between -1.0 and +1.5 which can be non-ionic, zwitterionic, cationic or anionic comprising, for example, groups $\alpha^1$, $\alpha^2$, $\alpha^3$, and $\alpha^4$ as defined below; S-linked groups including $S\alpha^1$, SCN, $SO_2\alpha^1$, $SO_3\alpha^1$, $SS\alpha1^1$, $SO\alpha^1$, $SO_2N\alpha^1\alpha^2$, $SN\alpha^1\alpha^2$, $S(N\alpha^1)\ \alpha^2$, $S(O)(N\alpha^1)\alpha^2$, $S\alpha^1(N\alpha^2)$, $SON\alpha^1\alpha^2$; O-linked groups including $O\alpha^1$, $OO\alpha^1$, OCN, $ON\alpha^1\alpha^2$; N-linked groups including $N\alpha^1\alpha^2$, $N\alpha^1\alpha^2\alpha^3+$, NC, $N\alpha^1O\alpha^2$, $N\alpha^1S\alpha^2$, NCO, NCS, $NO_2$, $N{=}N\alpha^1$, $N{=}NO\alpha^1$, $N\alpha^1CN$, $N{=}C{=}N\alpha^1$, $N\alpha^1N\alpha^2\alpha^3$, $N\alpha^1N\alpha^2N\alpha^3\alpha^4$, $N\alpha^1N{=}N\alpha^2$; other miscellaneous groups including COX, $CON_3$, $CON\alpha^1\alpha^2$, $CON\alpha^1CO\alpha^2$, $C({=}N\alpha^1)N\alpha^1\alpha^2$, CHO, CHS, CN, NC, and X.

$\alpha^1$, $\alpha^2$, $\alpha^3$, and $\alpha^4$ may be straight, branched or mono- or polycyclic aliphatic, mono or polyunsaturated alkyl, aryl, heteroalkyl, heteroaliphatic or heteroolefinic moiety comprising 3 to 150 carbon atoms together with 0-50 heteroatoms, especially O, N, S, P.

X is F, Cl, Br, or I.

H is hydrogen, O is oxygen, N is nitrogen, C is carbon, S is sulfur, Cl is chlorine, Br is bromine, I is iodine, F is fluorine.

Hammett sigma para values are discussed in Römpp Chemie Lexikon, Georg Thieme Verlag, Stuttgart, New York, 9th Edition, 1995 under "Hammett Gleichung".

Preferred polar functional substituents for use in the present invention as described include, but are not limited to, polyoxyalkylene (polyether), primary and secondary amine, amide, quaternary ammonium, carboxyl, sulfonate, sulfate, carbohydrate, phosphate, and hydroxyl. More preferably, the polar functional substituents of the present invention include, but are not limited to polyoxyalkylene, primary and secondary amine, amide and carboxyl.

Another highly prefereable polar functional substituents are amine-, polyol-type of the formula

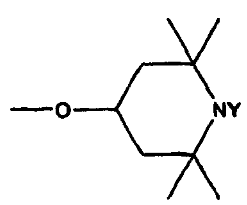

or

- $NYR^1$

wherein each $R^1$ is independently selected from the group consisting of a hydrogen atom and a group of formula $-R^2NY_2$, each Y is independently a hydrogen atom or Y', and each Y' is a group of formula

$-CH_2CH(OH)R^2-OH$

wherein $R^2$ is independently a divalent hydrocarbon group having 1 to 10 carbon atoms, and the proviso that every Y is not H.

More preferably $Y^1$ is a group of the formula $-CH_2CH(OH)CH_2OH$ and the functionalised silicone is of the pendant type, wherein n is from 200 to 500, p is from 20 to 50 and q, r and s are equal to zero.

Suitable functionalized silicones of the present invention include, but are not limited to, organomodified silicones with amine functionality available commercially under the trade names such as ADM1100 and ADM1600 from Wacker Silicones, DC2-8211, DC8822, DC8822A, DC8803, DC2-8040, DC2-8813, DC2-8630 and DC8566 from Dow Corning Corporation, KF-862, KF-861, KF-862S, KF-8005, KF-8004, KF-867S, KF-873, and X-52-2328 from Shin-Etsu Corporation, and TSF 4702, TSF 4703, TSF 4704, TSF 4705, TSF 4707, TSF 4708, TSF 4709, F42-B3115, SF 1708, SF 1923, SF 1921, SF 1925, OF TP AC3309, OF 7747, OF-NH TP AI3631, OF-NH TP AI3683 from GE Bayer Silicones.

Highly preferred functionalized silicones of the present invention are organomodified silicones with amine functionality with viscosities of greater than 4,000mPa.s which include, but are not limited to, commercially available fluids under the trade names ADM1100 from Wacker Silicones, DC8803 from Dow Corning Corporation, and TSF 4707 from GE Bayer Silicones.

According to a further aspect of the invention, a hair treatment kit is provided comprising:

(a) an oxidative bleaching composition
(b) a dye composition

a hair treatment composition as defined hereinabove comprised within component (a) and/or within component (b) and/or provided as a separate component.

The below table demonstrates the superior durability of the functional silicone fluids which may be incorporated into hair treatment compositions according to the present invention:

| Trade Name | Supplier | Viscosity (cp) | IFT | Durability Index |
|---|---|---|---|---|
| X22-3939A | Shin-Etsu | 3000 | ** | 0.0 |
| Wetsoft CTW | Wacker | 2800 | ** | 0.0 |
| Abilquat 3272 | Goldschmidt | 650 | ** | 0.0 |
| KF-945 | Shin-Etsu | 130 | 0.3 | 0.0 |
| Silwet L8500 | OSi | 250 | ** | 0.0 |
| KF905 | Shin-Etsu | 450 | ** | 0.0 |
| Y-14408 | OSi | 700 | ** | 0.0 |
| Abil Care 85 | Goldschmidt | 1900 | ** | 0.0 |
| Abilsoft AF100 | Goldschmidt | 300 | ** | 0.0 |
| XS69-B5476 | GE-Bayer | 1100 | ** | 0.1 |
| Abil B9950 | Goldschmidt | 80 | ** | 0.0 |
| Q2-8220 | Dow Corning | 200 | | |
| DC 2-8211 | Dow Corning | 900 | 1.9 | 1.1 |
| ADM 1600 | Wacker | 1650 | 2.2 | 1.8 |
| DC 8566 | Dow Corning | 2000 | 2.2 | 1.3 |
| ADM 656 | Wacker | 30 | 2.7 | 0.0 |
| KF-862 | Shin-Etsu | 750 | 3.5 | 1.3 |
| KF861 | Shin-Etsu | 3200 | 3.9 | 1.5 |
| KF860 | Shin-Etsu | 250 | 5 | 0.1 |
| DC2-8822 | Dow Corning | 2200 | 5.6 | 1.4 |
| Rhodorsil Fluid 21637 | Rhodia | 450 | 5.8 | 1.4 |

(continued)

| Trade Name | Supplier | Viscosity (cp) | IFT | Durability Index |
|---|---|---|---|---|
| ADM 1100 | Wacker | 5800 | 7 | 4.2 |
| ADM 652 | Wacker | 400 | 15 | 0.0 |
| BY-880 | Dow Corning | 2200 | 16 | 0.1 |
| X22-3701E | Shin-Etsu | 1700 | 21.5 | 0.0 |
| 1000 cst PDMS fluid | Dow Corning | 1000 | 32.9 | 0.0 |
| Abilquat 3474 | Goldschmidt | 8000 | ** | 0.0 |

** IFT too low too measure accurately via pendant drop.

The hair treatment composition according to the present invention may include a cosmetically acceptable vehicle to act as a diluent, dispersant, or carrier for the silicone oil in the composition, so as to facilitate the distribution of the silicone oil when the composition is applied. The vehicle may be an aqueous emulsion, water, liquid or solid emollients, solvents, humectants, propellants, thickeners and powders.

The hair treatment compositions according to the present invention is an oil-in-water emulsion (a silicone-in-water emulsion), and the functionalized silicone particle size is greater than 2$\mu$m.

The aqueous continuous phase of the emulsion treatment compositions of the present invention further comprises an emulsifier to facilitate the formation of the emulsion. Emulsifiers for use in the aqueous continuous phase of the present emulsion treatment compositions includes an anionic surfactant, cationic surfactant, amphoteric surfactant, water-soluble polymeric surfactant, water-soluble silicone-containing surfactant, nonionic surfactant having an HLB of greater than about 10, or a surfactant system capable of forming stabilizing liquid crystals around the silicone droplets. The nonionic surfactant preferably has an HLB of at least 12, and more preferably, an HLB value of at least about 15. Surfactants belonging to these classes are listed in McCutcheon's Emulsifiers and Detergents, North American and International Editions, MC Publishing Co., Glen Rock NJ, pages 235-246 (1993).

The emulsifier for the aqueous phase does not gel the aqueous phase. The emulsifier however forms a stabilizing layer of lamellar liquid crystals around silicone droplets. This barrier film prevents coalescence between emulsion droplets. For conciseness, the term "liquid crystal structure" as used herein, should be taken to also include gel networks, which are solidified liquid crystals. The surfactant system can be a single surfactant or a blend of surfactants. In some cases, a particular surfactant cannot form a liquid crystal structure alone, but can participate in the formation of liquid crystals in the presence of a second surfactant. Such a surfactant system forms a layer of lamellar liquid crystals around the silicone to provide a barrier between the silicone and the aqueous phase. This type of an emulsion is different from the conventional emulsions, which rely upon the orientation of the hydrophobic and hydrophilic components of a surfactant at an silicone-water interface. The formation of a layer of lamellar liquid crystals around the silicone can be detected by the presence of Maltese crosses viewed by optical microscopy through crossed polarizing plates or by freeze fracture electron microscopy.

Exemplary classes of surfactants capable of participating in the formation of a liquid crystal structure around the silicone droplets include, but are not limited to specific cationic surfactants, anionic surfactants, nonionic surfactants, quaternary ammonium surfactants.

Preferred surfactants for the formation of liquid crystals in the aqueous continuous phase are of the nonionic type and include $C_{16-20}$ fatty alcohols, and $C_{16-20}$ fatty alcohol ethoxylates with 1 to 30 ethylene oxide groups. Specific examples include cetearyl alcohol, cetyl alcohol, stearyl alcohol, arachidyl alcohol, oleyl alcohol, ceteareth ethoxylates with between 10 and 30 ethylene oxide groups, ceteth ethoxylates with between 10 to 30 ethylene oxide groups, steareth ethoxylates with between 10 and 30 ethoxylates, and combinations thereof. Preferably, $C_{16-22}$ fatty alcohols are used in combination with $C_{16-22}$ fatty alchol ethoxylates at a ratio of between 10:1 to 0.5:1, more preferably between 6:1 and 1:1, and most preferably between 5:1 and 1.5:1.

In the event that the surfactant system is intended to form liquid crystals, then the surfactant system advantageously does not comprise quaternary ammonium compounds of formula:

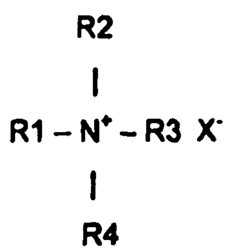

where R1 is an alkyl or alkenyl group having from about 8 to 22 carbon atoms, R2-R4 are each independently an alkyl or hydroxyalkyl group having from about 1 to 4 carbon atoms, and $X^-$ is a salt forming anion (e.g. chloride, bromide, acetate, alkylsulfate). Without wishing to be bound by theory, it is believed that mono-substituted quaternary ammonium molecules interact with the functionalized silicone droplets leading to a change of their interface with water, which in turn produces undesirable alterations in the silicone droplet sizes and its interfacial energy. All these effects make the functionalized silicone less compatible with fibres and thus non-durable. Accordingly, compositions according to the invention should be essentially free of such materials.

By contrast, when it is desired that the surfactant system form liquid crystals, then the composition will advantageously contain quaternary ammonium compounds of formula:

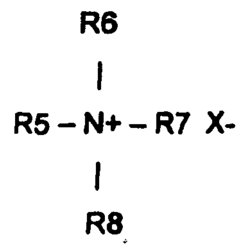

where R5, R6 are each independently an alkyl or alkenyl group having from about 8 to 22 carbon atoms, R7 is an alkyl or alkenyl group having from about 8 to 22 carbon atoms or alkyl or hydroxyalkyl group having from about 1 to 4 carbon atoms, R8 is an alkyl or hydroxyalkyl group having from about 1 to 4 carbon atoms, and X- is a salt forming anion (e.g. chloride, bromide, acetate, alkylsulfate).

Advantageously, in order to facilitate formation of liquid crystals, the surfactant system comprises amidoamines of the following general formula

R1 CONH (CH2)m N (R2)2

wherein R1 is a residue of C8 to C24 fatty acids, R2 is a C1 to C4 alkyl, and m is an integer from 1 to 4. Preferred amidoamine useful in the present invention includes stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and mixturesthereof; more preferably stearamidopropyldimethylamine, stearamidoethyldiethylamine, and mixtures thereof.

More advantageously, the amidoamines are partially quaternized with the acids selected from the group consisting of L-glutamic acid, lactic acid, hydrochloric acid, malic acid, succinic acid, acetic acid, fumaric acid, L-glutamicio acid hydrochloride, tartaric acid, and mixtures thereof; preferably L-glutamic acid, lactic acid, hydrochloric acid, and mixtures thereof. Preferably, the mole ratio of amidoamine to acid is from about 1:0.3 to about 1:1, more preferably from about 1:0.5 to about 1:0.

The presence of these materials in the composition may result in improved functionalized silicone deposition and durability. The preferred materials are di-alkyl substituted quaternary salts having alkyl chains containing 10-16 carbon atoms, and more preferably 14-16 carbon atoms, for example dicetyldimethyl ammonium chloride. Without wishing to be limited by theory, it is believed that di- and tri-substituted quaternary ammonium compounds form vesicle structures around the functionalized silicone droplets which act as a transport to deliver amino-silicone on the fibre surface without preventing desirable interactions of amino-silicone with the fibre.

The aqueous continuous phase comprises the emulsifier in an amount sufficient to stabilize the silicone. The aqueous continuous phase comprises the emulsifier in an amount of from about 0.1 % to about 15%, and more preferably from about 0.1% to about 10%, based on the weight of the aqueous continuous phase.

A physical property established to be important for achieving improved deposition and durability in the case of a surfactant system which forms liquid crystals is the particle size of the functionalized silicones of the present invention within the emulsion. The functionalised silicone will have a particle size greater than 2μm. Without wishing to be bound by theory, particle sizes below 500nm are believed to be too strongly emulsified, leading to poor deposition efficiency after the rinse process.

The topical cosmetic composition of the present invention may include optional benefit materials and cosmetic adjuncts, as long as the benefit materials or the adjuncts do not eliminate or substantially reduce the performance or shelf stability of the composition. The additional ingredients may include, for example dyes and coloring agents, fragrances; anionic, cationic, non-ionic, amphoteric or zwitterionic surfactants; buffers, masking fragrances, dispersing

agents, stabilizers, cationic polymers, perfumes, non-ionic polymers, anionic polymers, complex coacervates, complex coacervate capsules, metal salts, lewis acids, buffering agents, particulate thickeners, polymeric thickeners, wax thickeners, oils, emollients, humectants, moisturizers, pearlescents, opacifiers, enzymes, suspending agents, antimicrobials, preservatives, proteins, herb and plant extracts, bleach, peroxide, polyols, silicones, antibodies, pH adjusting agents including pH buffers, viscosity modifiers, preservatives, viscosity enhancers, gelling agents, chelators, oxidising agents, reducing agents, UV filters, emulsifying agents, moisturizing and conditioning agents, and other common adjuvants well known to those skilled in the art.

An antioxidant may also be incorporated within the emulsion treatment compositions. Suitable antioxidants include vitamin E and its derivatives, BHT and BHA.

In one embodiment of the present invention, a stabilizer comprising a polymeric thickener is employed. When polymeric thickeners are employed as the stabilizer in the emulsion treatment compositions herein, they are typically included in an amount ranging from about 0.01% to about 5%, preferably from about 0.3% to about 3%, based on the weight of the aqueous phase. The polymeric thickener is preferably an anionic, nonionic, cationic or hydrophobically modified polymer of natural, modified natural or synthetic origin from plants, microbials, animals or petroleum raw materials including karaya gum, tragacanth gum, gum arabic, gum ghatti, guar gum, locust bean gum, quince seed, psyllium seed, tamarind seed, carrageenan, alginates, agar, larch gum, pectins, starches, xanthan gum, dextran, casein, gelatin, keratin, shellac, cellulose derivatives, guar derivatives, acrylic acid polymers, polyacrylamides, and alkylene/alkylene oxide polymers. Preferred polymeric thickeners include guar gum, available commercially as SUPERCOL U, U NF, SUPERCOL GF, SUPERCOL G2S, and SUPERCOL G3 NF from Aqualon and JAGUAR GUM from Rhone-Poulenc; xanthan gum, available commercially as KELTROL CG, KELTROL CG F, KELTROL CG T, KELTROL CG TF, KELTROL CG 1000, KELTROL CG RD, KELTROL CG GM, KELTROL CG SF, from Calgon, ACCULYN 46,94 and 21 from Rohm & Haas and RHODICARE S, RHODICARE XC, RHODICARE H, AND RHODICARE D, from Rhone-Poulenc; hydroxyethylcellulose, available commercially as NATRASOL 210 types and NATRASOL 250 types from Aqualon; hydroxypropyl guar, available commercially as JAGUAR HP-8, JAGUAR HP-11, JAGUAR HP-60, and JAGUAR H-79 from Rhone-Poulenc. Additional specific polymeric thickeners that are suitable for the present invention are given in Rheological Properties of Cosmetics and Toiletries, edited by Dennis Laba, 1993, Marcel Dekker, Inc., pages 57 through 121 (ISBN 0-8247-9090-1).

Alternatively, the stabilizer employed can comprise $C_{10}$-$C_{22}$ ethylene glycol fatty acid esters. $C_{10}$-$C_{22}$ ethylene glycol fatty acid esters can also desirably be employed in combination with the polymeric thickeners hereinbefore described. The ester is preferably a diester, more preferably a $C_{14}$-$C_{18}$ diester, most preferably ethylene glycol distearate. When $C_{10}$-$C_{22}$ ethylene glycol fatty acid esters are utilized as the stabilizer in the emulsion treatment compositions herein, they are typically present in an amount of from about 3% to about 10%, preferably from about 5% to about 8%, more preferably from about 6% to about 8%, based on the weight of the aqueous phase.

The composition according to the present application finds particular utility in hair coloring compositions especially oxidative hair colorants wherein the hair is subjected to a particularly aggressive environment.

A preferred hair coloring agent for use herein is an oxidative hair coloring agent. The concentration of each oxidative hair coloring agent in the compositions according to the present invention may be from about 0.0001 % to about 5% by weight.

Any oxidative hair coloring agent can be used in the compositions herein. Typically, oxidative hair coloring agents comprise at least two components, which are collectively referred to as dye forming intermediates (or precursors). Dye forming intermediates can react in the presence of a suitable oxidant to form a colored molecule.

The dye forming intermediates used in oxidative hair colorants include: aromatic diamines, aminophenols, various heterocycles, phenols, napthols and their various derivatives. These dye forming intermediates can be broadly classified as; primary intermediates and secondary intermediates. Primary intermediates, which are also known as oxidative dye precursors, are chemical compounds which become activated upon oxidation and can then react with each other and/or with couplers to form colored dye complexes. The secondary intermediates, also known as color modifiers or couplers, are generally colorless molecules which can form colors in the presence of activated precursors/primary intermediates, and are used with other intermediates to generate specific color effects or to stabilise the color.

Primary intermediates suitable for use in the compositions and processes herein include: aromatic diamines, polyhydric phenols, amino phenols and derivatives of these aromatic compounds (e.g., N-substituted derivatives of the amines, and ethers of the phenols). Such primary intermediates are generally colorless molecules prior to oxidation.

While not wishing to be bound by any particular theory, it is believed that the process by which color is generated from these primary intermediates and secondary coupler compounds generally includes a stepwise sequence whereby the primary intermediate can become activated (by oxidation), and then enjoins with a coupler to give a dimeric, conjugated colored species, which in turn can enjoin with another 'activated' primary intermediate to produce a trimeric conjugated colored molecule.

In general terms, oxidative dye primary intermediates include those materials which, on oxidation, form oligomers or polymers having extended conjugated systems of electrons in their molecular structure. Because of the new electronic

structure, the resultant oligomers and polymers exhibit a shift in their electronic spectra to the visible range and appear colored. For example, oxidative primary intermediates capable of forming colored polymers include materials such as aniline, which has a single functional group and which, on oxidation, forms a series of conjugated imines and quinoid dimers, trimers, etc. ranging in color from green to black. Compounds such as p-phenylenediamine, which has two functional groups, are capable of oxidative polymerization to yield higher molecular weight colored materials having extended conjugated electron systems. Oxidative dyes known in the art can be used in the compositions according to the present invention. A representative list of primary intermediates and secondary couplers suitable for use herein is found in Sagarin, "Cosmetic Science and Technology"," Interscience, Special Ed. Vol. 2 pages 308 to 310.

The primary intermediates can be used alone or in combination with other primary intermediates, and one or more can be used in combination with one or more couplers. The choice of primary intermediates and couplers will be determined by the color, shade and intensity of coloration which is desired. There are nineteen preferred primary intermediates and couplers which can be used herein, singly or in combination, to provide dyes having a variety of shades ranging from ash blonde to black; these are: pyrogallol, resorcinol, p-toluenediamine, p-phenylenediamine, o-phenylenediamine, m-phenylenediamine, o-aminophenol, p-aminophenol, 4-amino-2-nitrophenol, nitro-p-phenylenediamine, N-phenyl-p-phenylenediamine, m-aminophenol, 2-amino-3-hydroxypyridine, 1-napthol, N,N bis (2-hydroxyethyl)p-phenylenediamine, diaminopyrazole, 4-amino-2-hydroxytoluene, 1,5-dihydroxynapthalene, 2-methyl resorcinol and 2,4-diaminoanisole. These can be used in the molecular form or in the form of peroxide-compatible salts.

The hair coloring compositions of the present invention may, in addition to or instead of an oxidative hair coloring agent, include non-oxidative and other dye materials. Optional non-oxidative and other dyes suitable for use in the hair coloring compositions and processes according to the present invention include both semi-permanent, temporary and other dyes. Non-oxidative dyes as defined herein include the so-called 'direct action dyes', metallic dyes, metal chelate dyes, fiber reactive dyes and other synthetic and natural dyes. Various types of non-oxidative dyes are detailed in: 'Chemical and Physical Behaviour of Human Hair' 3rd Ed. by Clarence Robbins (pp250-259); 'The Chemistry and Manufacture of Cosmetics'. Volume IV. 2nd Ed. Maison G. De Navarre at chapter 45 by G.S. Kass (pp841-920); 'cosmetics: Science and Technology' 2nd Ed., Vol. II Balsam Sagarin, Chapter 23 by F.E. Wall (pp 279-343); 'The Science of Hair Care' edited by C. Zviak, Chapter 7 (pp 235-261) and .'Hair Dyes', J.C. Johnson, Noyes Data Corp., Park Ridge, U.S.A. (1973), (pp 3-91 and 113-139).

The hair coloring compositions herein preferably comprise at least one oxidising agent, which may be an inorganic or organic oxidising agent. The oxidising agent is preferably present in the coloring composition at a level of from about 0.01% to about 10%, preferably from about 0.01% to about 6%, more preferably from about 1% to about 4% by weight of the composition.

A preferred oxidising agent for use herein is an inorganic peroxygen oxidising agent. The inorganic peroxygen oxidising agent should be safe and effective for use in the present compositions. Preferably, the inorganic peroxygen oxidising agents suitable for use herein will be soluble in the compositions according to the present invention when in liquid form or in the form intended to be used. Preferably, inorganic peroxygen oxidising agents suitable for use herein will be water-soluble. Water soluble oxidising agents as defined herein means agents which have a solubility to the extent of about 10g in 1000ml of deionised water at 25°C ("Chemistry" C. E. Mortimer. 5th Edn. p277).

The inorganic peroxygen oxidising agents useful herein are generally inorganic peroxygen materials capable of yielding peroxide in an aqueous solution. Inorganic peroxygen oxidising agents are well known in the art and include hydrogen peroxide, inorganic alkali metal peroxides such as sodium periodate, sodium perbromate and sodium peroxide, and inorganic perhydrate salt oxidising compounds, such as the alkali metal salts of perborates, percarbonates, perphosphates, persilicates, persulphates and the like. These inorganic perhydrate salts may be incorporated as monohydrates, tetrahydrates etc. Mixtures of two or more of such inorganic peroxygen oxidising agents can be used if desired. While alkali metal bromates and iodates are suitable for use herein the bromates are preferred. Highly preferred for use in the compositions according to the present invention is hydrogen peroxide.

The compositions herein may instead or in addition to the inorganic peroxygen oxidising agent(s), comprise one or more preformed organic peroxyacid oxidising agents.

Suitable organic peroxyacid oxidising agents for use in the coloring compositions according to the present invention have the general formula:

$$R-C(O)OOH$$

wherein R is selected from saturated or unsaturated, substituted or unsubstituted, straight or branched chain, alkyl, aryl or alkaryl groups with from 1 to 14 carbon atoms.

The organic peroxyacid oxidising agents should be safe and effective for use in the compositions herein. Preferably, the preformed organic peroxyacid oxidising agents suitable for use herein will be soluble in the compositions used according to the present invention when in liquid form and in the form intended to be used. Preferably, organic peroxyacid oxidising agents suitable for use herein will be water-soluble. Water-soluble preformed organic peroxyacid

oxidising agents as defined herein means agents which have a solubility to the extent of about 10g in 1000ml of deionised water at 25°C ("Chemistry" C. E. Mortimer. 5th Edn. p277).

The compositions herein may optionally contain a transition metal containing catalyst for the inorganic peroxygen oxidising agents and the optional preformed peroxy acid oxidising agent(s). Suitable catalysts for use herein are disclosed in WO98/27945.

The compositions herein may contain as an optional component a heavy metal ion sequestrant. By heavy metal ion sequestrant it is meant herein components which act to sequester (chelate or scavenge) heavy metal ions. These components may also have calcium and magnesium chelation capacity, but preferably they show selectivity to binding heavy metal ions such as iron, manganese and copper. Such sequestering agents are valuable in hair coloring compositions as herein described for the delivery of controlled oxidising action as well as for the provision of good storage stability of the hair coloring products.

Heavy metal ion sequestrants may be present at a level of from about 0.005% to about 20%, preferably from about 0.01% to about 10%, more preferably from about 0.05% to about 2% by weight of the compositions.

Suitable sequestering agents are disclosed in WO98/27945.

For use, the hair treatment compositions according to an embodiment of the invention may be provided at a pH from about 3 to 11, preferably from 4 to 10.5.

The hair treatment compositions according to the present invention may be provided in any suitable physical form, for example as low to moderate to high viscosity liquids, lotions, milks, mousses, dispersions, sprays, gels, foams, aerosols, and creams. These compositions may be produced by procedures well known to the skilled artisan. The compositions may be incorporated into various products, including but not limited to, rinse-off and leave-on products such as hair shampoos, skin cleansers, skin lotions, hair conditioners, hair dyes, after colorant conditioners, hair permanent waves, hair straighteners, hair bleaches, styling sprays, hair mousses and two-in-one shampoos.

The hair treatment compositions of the present invention can be formulated as a fluid, lotion, fluid cream or cream having a viscosity from 500 to 100,000 mPa.s or above. The compositions can be packaged in a suitable container to suit its viscosity and intended use by the consumer. For example, a lotion or fluid cream can be packaged in a bottle, a roll-ball applicator, a propellant-driven aerosol device, a container fitted with a pump suitable for hand or finger operation, or the like. When the composition is a cream, it can simply be stored in a non-deformable bottle or squeeze container, such as a tube or a lidded jar.

The hair treatment compositions of the present invention can be applied to wet hair, partially wet hair or dry hair. If desired, the compositions can be mixed with additional water or separate composition prior to or during application to the hair. The contact time between the emulsion treatment compositions of the present invention and the substrate can vary between a few seconds and about 1 hour, preferably between 10 seconds and 50 minutes, more preferably between 30 seconds and 40 minutes. The composition may be thoroughly rinsed from the hair, or the compositions can be applied as a leave-on product, as desired.

## TEST METHODS

### Interfacial tension measurement protocol

The silicone/water interfacial tensions of the organomodified silicones were measured via pendant drop shape analysis on a Kruss DSA-10 instrument as taught in F. K. Hansen, G. Rodsrun, "Surface tension by pendant drop. A fast standard instrument using computer image analysis", Journal of Colloid and Interface Science, Volume 141, Issue 1, January 1991, pages 1-9. The accuracy of this method is dependent upon the density difference between the reference fluid (usually water) and the test fluid. Given that many of the present functionalized silicones have densities approaching that of water, $D_2O$ (with a density of 1.1 g/cm$^{-3}$) was substituted for $H_2O$ as the more dense phase, in order to ensure a sufficient density difference. The respective densities of the organomodified silicones were measured with a Calculating Precision Density Meter DMA 55 instrument from Apollo Scientific Limited.

### Viscosity of functionalized silicone fluids - measurement protocol

An AR 500 rotational rheometer (TA Instruments Ltd., Leatherhead, Surrey KT22 7UQ, UK) is used to determine the viscosity of the functionalized silicone fluids used herein. The determination is performed at 30°C, with the 4cm 2° steel cone measuring system set with a 49μm (micron) gap and is performed via the programmed application of a shear stress of 0.5 to 590 Pa over a 2 minute time period. These data are used to create a shear rate vs. shear stress curve for the material. This flow curve can then be modelled in order to provide a material’s viscosity. These results were fitted with the following well-accepted Newtonian model:

$$\text{Viscosity}, \ \mu = \sigma/\gamma$$

(where o is shear stress; y is shear rate)

Method for assessing silicone particle size within the oil-in-water emulsion

The following method had to be adopted in order to distinguish between the silicone particles and other aspects of the emulsion, such as the lamellar gel phase.

Sample Preparation:

A small drop of the sample product is placed on a standard microscope slide; either side of the droplet is placed a standard cover slip upon which a third cover slip is placed directly above the droplet and hence bridging the other two cover slips. The third cover slip is then pressed down until it contacts the other two cover slips - thereby trapping product. The sample thickness is therefore guaranteed to always be the same (namely the thickness of the standard cover slip) and given this is larger than the diameter of particles reduces the chances of sample preparation affecting (deforming) the particles. The preferred sample amount is thus that defined by the volume of the void under said third cover slip (too little and the sample will not contact the underside of said third cover slip, too much and the sample will ooze from the side on compression of said third cover slip).

Particle Size Measurement:

The particle size method is typical of those known in the art, and utilizes a standard Nikon optical microscope, with standard transmitted light using x10 objective. To aid accuracy, a Lucia G software (by Nikon) is used with the following procedure. The first step of analysis requires the user to scan and select a field that is representative of the bulk - this typically requires multiple preparations for accuracy. The observed image is transmitted via JVC video camera to a standard monitor and each particle is measured by using the standard Measure macro; namely, clicking on each side of the particle - hence measuring a diameter. To account for none spherical particles, the 'diameter' is always assessed horizontally across the monitor. By measuring in one plane, the technique automatically compensates for non spherical geometry and due to the large number of particles measured results in an equivalent average diameter. Although equivalent diameters may be determined by measuring the major and minor axes and calculating equivalent diameter via aspect ratio equations, the above technique provides equally accurate results.

Since it is typical human nature to count the largest particles first and thus to ensure that all particles are counted and measured, a small (typically using an erasable pen) dot should be placed on the monitor over each counted particle. The count procedure is continued until every single visible particle is counted within the field. In the case of a very small particle size distribution, this may result in over 400 counts. In the case of larger particle sizes, one might expect approximately 100 counts per field, however in such cases additional fields would be selected to ensure at least 200 separate particles are counted. In summary, in all cases at least 200 separate particles should be measured and in all cases all particles (in practice the upper limit being 400-500) in one field are counted. On average, across all the examples sighted herein, about 300 particles would be measured per sample. Analysis can be as below (standard volume average calculated by hand to demonstrate the technique) or, more typically, using the macro. that automatically sorts the data reporting a volume average (assuming a spherical geometry based on the diameter measured above).

**Silicone Durability Index Method**

Hair substrate preparation

Durability is assessed on a polar, chemically damaged substrate. Hair is supplied by Hugo Royer International Limited (10 Lakeside Business Park, Sandhurst, Berkshire, GU47 9DN, England) and is a blended, Eastern European, mid-brown human hair. Prior to use the hair is assessed and qualified for low cuticular damage (<20%) and misalignment (<5%), based on at least 200 hair strands per batch. Any damage on a hair strand counts as one point damaged, and then the total is calculated as a percentage. This hair is made into 4" 2 g round tied switches (where the length and weight of hair corresponds to the hair below the tie). To obtain a damaged, polar hair substrate the following protocol is used.

Hair switches are chemically damaged using the following two component bleaching formulations:

| Peroxide base | |
|---|---|
| Ingredients | Wt/Wt% |
| 1. Emulsion base: | |
| Deionized water | 29.78 |
| Cetyl alcohol (1) | 2.24 |
| Stearyl alcohol(2) | 2.24 |
| Ceteareth-25 (3) | 1.50 |
| Phenoxyethanol (4) | 0.11 |
| Sodium benzoate (5) | 0.09 |
| Tetrasodium EDTA (87%) (6) | 0.04 |
| 2. Chelant premix | |
| Deionized water | 35.72 |
| Pentasodium pentetate (40%) (7) | 0.24 |
| Hydroxyethane diphosphonic acid (60%)(8) | 0.16 |
| Phosphoric acid (75%) (9) | 0.08 |
| Sodium stannate (95%)(10) | 0.04 |
| 3. Peroxide mix | |
| Hydrogen peroxide (35%) (11) | 17.15 |
| Deionized water | 10.61 |

| Carrier base for dye base | |
|---|---|
| Ingredients | Wt/Wt% |
| 1. Acetic acid pre-mix | |
| Deionized water | 46.49 |
| Acetic acid (50%) (12) | 3.91 |
| 2. Emulsion base | |
| Deionized water | 29.78 |
| Cetyl alcohol (1) | 2.24 |
| Stearyl alcohol (2) | 2.24 |
| Ceteareth-25(3) | 1.50 |
| Phenoxyethanol (4) | 0.11 |
| Sodium benzoate (5) | 0.09 |
| Tetrasodium EDTA (87%) (6) | 0.04 |
| Ammonium hydroxide (13) | 13.60 |
| (1): available as Surfac cetyl alcohol from Surfachem, Leeds, UK | |
| (2): available as Surfac stearyl alcohol from Surfachem, Leeds, UK | |
| (3): available as Volpo CS25 from Croda, North Humberside. UK | |
| (4): available as Phenoxyethanol from Nipa-Hardwicke, Wilmington, Delaware | |
| (5): available as Sodium benzoate EP/USP from Haltermann, Cumbria, UK | |
| (6): available as Edeta B powder from BASF, Cheadle, Cheshire, UK | |
| (7): available as Trilon C liquid from BASF, Cheadle, Cheshire, UK | |
| (8): available as Dequest 2010 from Solutia, Newport, South wales | |
| (9): available as Phosphoric acid 750F from Albright & Wilson, West Midlands, UK | |
| (10): available as Sodium stannate, Aldrich | |
| (11): available as Hydrogen peroxide 35% 171/4 from Ellis & Everard, Walsall, UK | |
| (12): available as 50% acetic acid from Hays, Greenwich, London, UK | |
| (13): available as Ammonium Solution BP grade from Brotherton Speciality Products, West Yorkshire, UK | |

These products are made using the flowing protocols:

Peroxide base:

The first stage is to make the emulsion base; this is prepared by adding to a vessel deionized water and commencing agitation, and then heating to 82 °C.

Then tetrasodium EDTA and sodium benzoate are added and dissolved, followed by addition of ceteareth25, cetyl alcohol and stearyl alcohol. During the addition process the temperature is maintained above 80 °C, finally phenoxyethanol is added, the mixture is then homogenized for 30 min The emulsion structure is obtained by cooling whilst still high shear mixing the product down below 50 °C. The emulsion base is then left to thicken for 60 min.

The chelants are added to the deionised water with mixing to form the chelant premix. This is then added with stirring to the pre-made emulsion base. Adding the peroxide mix water followed by hydrogen peroxide to the emulsion base/chelant premix and stirring until homogeneous makes the completed peroxide base.

Carrier base for dyes

The carrier base for dyes is prepared by adding water to a vessel and commencing agitation, followed by the addition of acetic acid, then the emulsion base (see emulsion base preparation described hereinbefore for the peroxide base). When fully mixed, ammonium hydroxide is added to the mixture and the stirring continued until the product is homogenous.

To use this bleaching system, equal weights of the two components, the peroxide base and carrier base for dyes are mixed together thoroughly. To each dry untreated hair switch, 4 g of this bleaching system is then applied, and thoroughly worked into the hair, using the fingers, to ensure even, complete, coverage. The hair switch is then wrapped in cling film and incubated in an oven at 30 °C for 30 minutes, after which the product is rinsed for 2 minutes (in a sink fitted with a shower attachment set with a flow rate of 6 ± 1 L $min^{-1}$ and a temperature of 37 ± 2 °C ) with finger agitation. Finally the switches are dried using a hot air drier (Babyliss Lightweight Professional model 1015 (1400 W)) for 3 min. The bleached hair switches are then washed in a sink fitted with a shower attachment set with a flow rate of 6 ± 1 L $min^{-1}$ and a temperature of 37 ± 2 °C. Switches are initially wetted under the shower attachment for 30 s. The hair is then removed from the water flow and 0.2 g of shampoo (Pantene Clarifying Shampoo) is applied down each switch, and then lathered for 30 s by hand before rinsing for 60 s under the shower. The hair is again removed from the shower, and has a further 0.2 g of shampoo applied, and lathered for 30 s before finally rinsing under the shower for 60 s. Hair switches are then dried using a hot air drier (Babyliss Lightweight Professional model 1015 (1400 W)) for 3 min. This washing protocol comprising two shampoo applications and one drying step is defined as a single wash cycle. This washing method is then repeated again through another complete wash cycle. The dry hair switches are then bleached again according to the method outlined above and subsequently washed again through 2 complete wash cycles. This hair is hereinafter defined as "damaged" hair and is hereinafter used as a hydrophilic hair substrate.

Hair treatment

Silicones are deposited on to the hair via a solvent matrix. Propan-2-ol (obtained from Aldrich Chemicals, product # 15,479-2) is used as the solvent in the delivery of functional silicone fluids herein. The silicone fluid is solubilized in 2-propanol at a concentration of 0.20 % using a magnetic stirrer. Hair tresses are laid flat on cling film and the resulting 2-propanol/silicone solution applied using a syringe at a dosage of 1g silicone solution/ 1g of hair (half to each side). The solution is then massaged into the hair using fingers for 30 s. The treated switches are allowed to dry naturally in the ambient atmosphere. When the switches are dry they are split into two groups both comprising equal numbers of damaged hair switches. The first are used to measure the initial deposition after the 2-propanol deposition. The second set is washed to assess the silicone durability. The hair switches are washed in a sink fitted with a shower attachment set with a flow rate of 6 ± 1 L $min^{-1}$ and a temperature of 37 ± 2 °C. Switches are initially wetted under the shower attachment for 30 s. The hair is removed from the water flow and 0.2 g of shampoo ("Pantene Classic Clean Shampoo") is applied along each switch, and then lathered for 30 s by hand before rinsing for 60 s under the shower. The switch then has a further 0.2 g of shampoo application, and is lathered for 30 s before finally rinsing under the shower for 60 s. Hair switches are then dried using a hot air drier (Babyliss Lightweight Professional model 1015 (1400 W)) for 3 min. This protocol comprising two shampoo applications and one drying step is defined as one complete wash cycle. This washing protocol is then repeated again through another eleven complete cycles (to make twelve wash cycles in total). These switches are then measured for silicone deposition to assess the durability performance.

Deposition measurement

A wavelength dispersive X-Ray Fluoresence spectrometer (Phillips Electronics, PW2404 Sequential "4000W" X-Ray Spectrometer System) is utilised to determine the silicone deposition level on hair. The spectrometer is fitted with a Rhodium tube and includes an InSb crystal to facilitate high sensitivity silicone detection.

Characteristic X-Ray photons are produced from the ejection of an inner shell electron of an silicone atom followed by a transition of an electron from a higher energy state to the empty inner shell. X-Ray fluorescence of silicon in polydimethylsiloxane (PDMS) is directly proportional to the amount of PDMS deposited on the hair. A critical component to facilitate the use of XRF technology is the ability to present the sample to the spectrometer in a consistent manner. The hair switch is arranged in a custom-made sample holder, which presents a continuous, flat, aligned hair surface across the exposed sample area (16 mm diameter). The sample is analysed under a helium atmosphere using a Tube voltage of 32 kV and current of 125 mA, with an irradiation/acquisition time of 60 s.

The drift in the analytical signal is regularly monitored and evaluated. The preferred approach employed is to use a known standard that does not need to be prepared each time the drift is assessed. An Ausmon sample is an appropriate monitor sample for many applications, including silicon determinations. A drift correction with the Ausmon sample for silicon is performed at the beginning of each day samples are analyzed. The calculated drift is below 3% between sets of analysis.

Calculation of the amount of silicon on hair in units of ppm from can be made with equation 1.

$$x_2=(I-b_1)/m_1 \quad (1)$$

Where $m_1$ and $b_1$ are calculated from a calibration curve constructed from measurements of the XRF signal as a function of the amount of silicone deposited on hair subsequently assayed using atomic absorption on the extracted silicone.

To translate the XRF silicone deposition data obtained as hereinbefore described into a measure of silicone durability, it is necessary to generate a silicone durability index value. To generate the silicone durability index value the following equation is employed:

$$\text{Silicone durability index value} = \frac{Dep(12cycle)}{Dep(initial)}$$

Where Dep(initial) equals the XRF deposition value obtained on hair after silicone deposition with no washing cycles, Dep(12cycles) equals the XRF deposition value obtained on hair after silicone deposition and subsequent 12 wash cycles.

EXAMPLES

The following examples further describe and demonstrate the preferred embodiments within the scope of the present invention. The examples are given solely for the purpose of illustration, and are not to be construed as limitations of the present invention since many variations thereof are possible without departing from its scope.

Examples 1-3 - Colorant compositions (not part of claimed subject-matter)

| Peroxide base | #1 | #2 | #3 |
|---|---|---|---|
| Ingredients<br>Emulsion base: | Wt% | Wt% | Wt% |
| Deionized water | 29.17 | 29.17 | 29.17 |
| Cetyl alcohol (1) | 2.20 | 2.20 | 2.20 |
| Stearyl alcohol (2) | 2.20 | 2.20 | 2.20 |
| Ceteareth-25 (3) | 1.47 | 1.47 | 1.47 |
| Phenoxyethanol (4) | 0.11 | 0.11 | 0.11 |
| Sodium benzoate (5) | 0.09 | 0.09 | 0.09 |

(continued)

| Peroxide base | #1 | #2 | #3 |
|---|---|---|---|
| Ingredients<br>Emulsion base: | Wt% | Wt% | Wt% |
| Tetrasodium EDTA (87%)(6) | 0.04 | 0.04 | 0.04 |
| Deionized water | 35.00 | 35.00 | 35.00 |
| Pentasodium pentetate (40%) (7) | 0.24 | 0.24 | 0.24 |
| Hydroxyethane diphosphonic acid (60%) (8) | 0.16 | 0.16 | 0.16 |
| Phosphoric acid (75%) (9) | 0.08 | 0.08 | 0.08 |
| Sodium stannate (95%) (10) | 0.04 | 0.04 | 0.04 |
| Hydrogen peroxide (35%) (11) | 16.80 | 16.80 | 16.80 |
| Deionized water | 10.40 | 10.40 | 9.40 |
| - Aminofunctional polydimethylsiloxane sold under the name Wacker-belsil®ADM1100 by the company Wacker | 0 | 2.00 | 0 |
| - Aminofunctional polydimethylsiloxane sold under the name DC 8803silicone fluid by the company Dow Corning | 2.00 | 0 | 3.00 |

| Carrier base for dye base | #1 | #2 | #3 |
|---|---|---|---|
| Ingredients | Wt% | Wt% | Wt% |
| Deionized water | 46.49 | 46.49 | 46.49 |
| Acetic acid (50%) (12) | 3.91 | 3.91 | 3.91 |
| Emulsion base (see ingredients above) | 36.00 | 36.00 | 36.00 |
| Ammonium hydroxide (13) | 13.60 | 13.60 | 13.60 |

(1): available as Surfac cetyl alcohol from Surfachem, Leeds, UK
(2): available as Surfac stearyl alcohol from Surfachem, Leeds, UK
(3): available as Volpo CS25 from Croda, North Humberside. UK
(4): available as Phenoxyethanol from Nipa-Hardwicke, Wilmington, Delaware
(5): available as Sodium benzoate EP/USP from Haltermann, Cumbria, UK
(6): available as Edeta B powder from BASF, Cheadle, Cheshire, UK
(7): available as Trilon C liquid from BASF, Cheadle, Cheshire, UK
(8): available as Dequest 2010 from Solutia, Newport, South wales
(9): available as Phosphoric acid 750F from Albright & Wilson, West Midlands, UK
(10): available as Sodium stannate, Aldrich
(11): available as Hydrogen peroxide 35% 171/4 from Ellis & Everard, Walsall, UK
(12): available as 50% acetic acid from Hays, Greenwich, London, UK
(13): available as Ammonium Solution BP grade from Brotherton Speciality Products, West Yorkshire, UK

Production of the example colorant applications

Peroxide base:

The emulsion base is prepared by adding to a vessel the deionized water and commencing agitation with heating to 82°C. Then the preservatives (tetrasodium EDTA, sodium benzoate) are added and dissolved. This is followed by addition of ceteareth25, cetyl alcohol and stearyl alcohol while keeping the temperature above 80°C. Then phenoxytol is added. The mixture is then fully blended hot through a recirculation line and homogenized. The emulsion structure is obtained by cooling the product down below 50°C and shearing while cooling. The product is left to thicken for 60min.

The chelant premix is prepared by adding the chelants to water and mixing them together in a vessel. Then

this solution is added to the emulsion base. The completed peroxide base is made by adding water to the previous mixture followed by the hydrogen peroxide while stirring.

To this peroxide base the silicone can then be added and stirred.

Carrier system for dye base :

The carrier base is prepared by adding water to a vessel and commencing agitation, followed by the addition of acetic acid. Then emulsion base (see emulsion base preparation described above) is added. When fully homogenized, ammonium hydroxide is added to the mixture.

Examples 4-5 - After colorant conditioners (not part of claimed subject-matter)

| | #4 | #5 |
|---|---|---|
| Ingredients | Wt% | Wt% |
| Deionized water | 61.95 - qs | 60.95 - qs |
| Emulsion base: | | |
| Deionized water | 29.76 | 29.76 |
| Cetyl alcohol (1) | 2.25 | 2.25 |
| Stearyl alcohol (2) | 2.25 | 2.25 |
| Ceteareth-25 (3) | 1.50 | 1.50 |
| Phenoxyethanol (4) | 0.11 | 0.11 |
| Sodium benzoate (5) | 0.09 | 0.09 |
| Tetrasodium EDTA (87%) (6) | 0.04 | 0.04 |
| Citric acid anhydrous fine (14) | pH trim | pH trim |
| - Aminofunctional polydimethylsiloxane sold under the name Wacker-belsil®ADM1100 by the company Wacker | 2.00 | 0 |
| - Aminofunctional polydimethylsiloxane sold under the name DC 8803silicone fluid by the company Dow Corning | 0 | 3.00 |
| (14): available as citric acid anhydrous fine from Aldrich | | |

**Composition preparation**

The conditioner composition is prepared by adding to a vessel the deionized water and the emulsion base (see emulsion base preparation described above) while stirring. When homogenized citric acid is added to the mixture until the pH of the emusltion is between 5 and 6.

Then the single fluids can then be added to the emulsion and stirred.

Examples 6-9: Hair Conditioner

| | #6 | #7 | #8 | #9 |
|---|---|---|---|---|
| Ingredients | Weight percent | Weight percent | Weight percent | Weight percent |
| | | | | |
| Stearamidopropyl dimethylamine[1] | 1.20 | 1.60 | 2.00 | 2.00 |
| L- Glutamic acid[2] | 0.38 | 0.51 | 0.64 | 0.64 |
| Dicetyl dimethyl ammonium chloride[8] | - | - | - | 0.50 |
| Cetyl Alcohol (95% pure) [3] | 4.50 | 3.60 | 4.50 | 4.50 |

(continued)

| | #6 | #7 | #8 | #9 |
|---|---|---|---|---|
| Ingredients | Weight percent | Weight percent | Weight percent | Weight percent |
| Stearyl Alcohol (95% pure) [3] | 2.50 | 2.00 | 2.50 | 2.50 |
| Amino-silicone[9] | 5.00 | 5.00 | | 5.00 |
| Amino-, polyol-silicone[10] | | | 5.00 | |
| Kathon CG[4] | 0.03 | 0.03 | 0.03 | 0.03 |
| Benzyl alcohol[5] | 0.40 | 0.40 | 0.40 | 0.40 |
| EDTA BS[6] | 0.10 | 0.10 | 0.10 | 0.10 |
| Perfume[7] | 0.25 | 0.25 | 0.25 | 0.25 |
| Water | 85.64 | 86.51 | 84.58 | 84.08 |

1 - supplied by Inolex under trade name Lexamine S-13
2 - supplied by Ajinomoto Ltd
3 - supplied by Procter & Gamble
4 - supplied by Rohm & Haas
5 - supplied by Haarman & Reimer
6 - supplied by BASF
7 - supplied by Firmenich
8 - supplied by Goldschmidt under trade name Varisoft 432CG
9 - supplied by Wacker under trade name ADM1100
10- amino-, polyol- functional silicone supplied by Dow Corning Corporation, reference number 17828-137.

In examples 6 to 9, water, dicetyl dimethyl ammonium chloride, stearamidopropyl dimethylamine and L-glutamic acid are mixed at temperature above 70°C. Then cetyl alcohol, stearyl alcohol and benzyl alcohol are added with agitation. After cooling down below 60°C, amino-silicone, Kathon CG and perfume are added with agitation, and then cooled down to about 30°C.

## Claims

1. Hair treatment composition in the form of an oil in water emulsion comprising a functionalized silicone polymer having an interfacial tension (IFT) of 1 to 12 mN/m and a viscosity from 400 to 150000 mPa.s at 30°C, wherein the functionalized silicone polymer deposits durably on hair, and wherein said silicone polymer has a particle size greater than 2 $\mu$m, wherein the hair treatment composition additionally comprises 0.1 to 15% based on the weight of the aqueous continuous phase of emulsifier, wherein the emulsifier comprises a surfactant system including one or more of an anionic surfactant, cationic surfactant, amphoteric surfactant, water-soluble polymeric surfactant, water soluble silicone-containing surfactant and a non-ionic surfactant, wherein the surfactant system comprises an amidoamine according to the formula $R_1CONH(CH_2)_mN(R_2)_2$ wherein $R_1$ is a residue of $C_8$ to $C_{24}$ fatty acids, $R_2$ is $C_1$ to $C_4$ alkyl and m is an integer from 1 to 4 and wherein the surfactant system is capable of forming a liquid crystal structure around the silicone droplets.

2. Hair treatment composition according to claim 1, wherein the IFT is in the range 1 to 10 mN/m, preferably in the range 1 to 8 mN/m, most preferably in the range 1 to 4 mN/m.

3. Hair treatment composition according to any one of the preceding claims, wherein the functionalized silicone has a viscosity in the range 4000 to 25,000 mPa.s at 30°C.

4. Hair treatment composition according to any one of the preceding claims, wherein the functionalized silicone is present in an amount ranging from 0.1 to 20%wt, preferably from 0.5 to 7.5%wt.

5. Hair treatment composition according to claim 1, wherein the surfactant system does not comprise quaternary

ammonium compounds of formula:

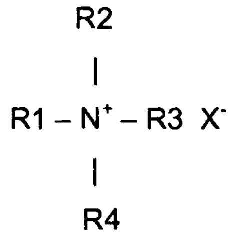

where R1 is an alkyl or alkenyl group having from about 8 to 22 carbon atoms, R2-R4 are each independently an alkyl or hydroxyalkyl group having from about 1 to 4 carbon atoms, and $X^-$ is a salt forming anion.

**6.** Hair treatment composition according to claim 1 or 5, wherein the surfactant system comprises quaternary ammonium compounds of formula

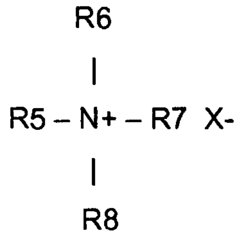

where R5, R6 are each independently an alkyl or alkenyl group having from about 8 to 22 carbon atoms, R7 is an alkyl or alkenyl group having from about 8 to 22 carbon atoms or alkyl or hydroxyalkyl group having from about 1 to 4 carbon atoms, R8 is an alkyl or hydroxyalkyl group having from about 1 to 4 carbon atoms, and X- is a salt forming anion.

**7.** Hair treatment composition according to any one of the preceding claims, wherein the functionalized silicone is an organomodified silicone of the pendant or graft type according to the following formula:

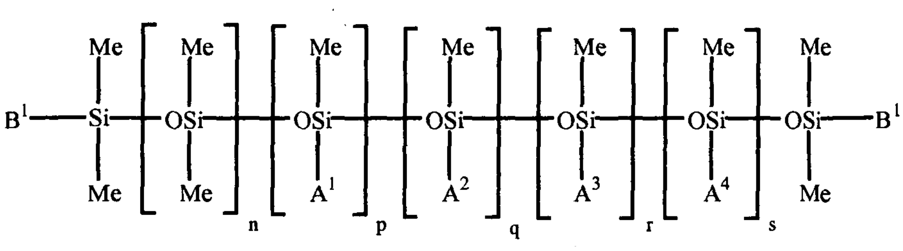

or a block copolymer type according to the following formula:

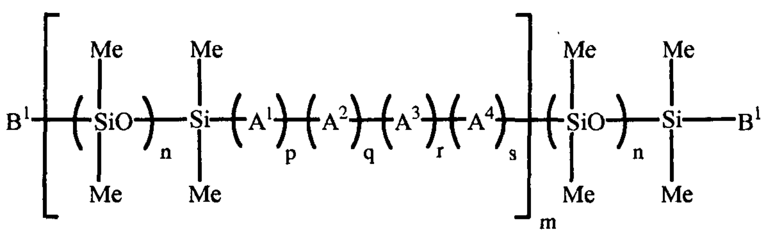

where m is greater than or equal to 1, n is about 50 to 2000, p is about 0 to 50, q is about 0 to 50, r is about 0 to 50, s is about 0 to 50, wherein p + q + r + s is greater than or equal to 1, $B^1$ is H, OH, an alkyl or an alkoxy group; $A^1$, $A^2$, $A^3$ and $A^4$ are be straight, branched or mono- or polycyclic aliphatic, mono or polyunsaturated alkyl, aryl, heteroalkyl, heteroaliphatic or heteroolefinic moiety comprising 3 to 150 carbon atoms together with 0-50 heteroatoms

including one or more polar substituents selected from electron withdrawing, electron neutral, or electron donating groups with Hammett sigma para values between -1.0 and +1.5.

**8.** Hair treatment composition according to claim 7, wherein the polar substituents comprise groups $\alpha^1$, $\alpha^2$, $\alpha^3$, and $\alpha^4$; S-linked groups including $S\alpha^1$, SCN, $SO_2\alpha^1$, $SO_3\alpha^1$, $SS\alpha 1^1$, $SO\alpha^1$, $SO_2N\alpha^1\alpha^2$, $SN\alpha^1\alpha^2$, $S(N\alpha^1)\ \alpha^2$, $S(O)(N\alpha^1)\ \alpha^2$, $S\alpha^1(N\alpha^2)$, $SON\alpha^1\alpha^2$; O-linked groups including $O\alpha^1$, $OO\alpha^1$, OCN, $ON\alpha^1\alpha^2$; N-linked groups including $N\alpha^1\alpha^2$, $N\alpha^1\alpha^2a^3+$, NC, $N\alpha^1O\alpha^2$, $N\alpha^1S\alpha^2$, NCO, NCS, $NO_2$, $N{=}N\alpha^1$, $N{=}NO\alpha^1$, $N\alpha^1CN$, $N{=}C{=}N\alpha^1$, $N\alpha^1N\alpha^2\alpha^3$, $N\alpha^1N\alpha^2N\alpha^3\alpha^4$, $N\alpha^1N{=}N\alpha^2$; COX, $CON_3$, $CON\alpha^1\alpha^2$, $CON\alpha^1CO\alpha^2$, $C({=}N\alpha^1)N\alpha^1\alpha^2$, CHO, CHS, CN, NC, and X, where:

$\alpha^1$, $\alpha^2$, $\alpha^3$, and $\alpha^4$ may be straight, branched or mono- or polycyclic aliphatic, mono or polyunsaturated alkyl, aryl, heteroalkyl, heteroaliphatic or heteroolefinic moiety comprising 3 to 150 carbon atoms together with 0-50 heteroatoms, especially O, N, S, P, and
X is F, Cl, Br, or I, where
H is hydrogen, O is oxygen, N is nitrogen, C is carbon, S is sulfur, Cl is chlorine, Br is bromine, I is iodine, F is fluorine.

**9.** Hair treatment composition according to claim 8, wherein the polar substituents are selected from polyoxyalkylene, primary and secondary amine, amide, quaternary ammonium, carboxyl, sulfonate, sulfate, carbohydrate, phosphate, and hydroxyl and mixtures of these.

**10.** Hair treatment composition according to claim 9, wherein the polar substituents comprise amine substituents.

**11.** Hair treatment composition according to claim 8, wherein the polar substituents comprise amino-, polyol- substituents of the formula:

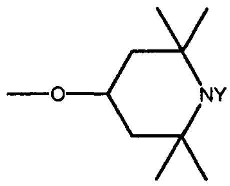

or

$-NYR^1$

wherein each $R^1$ is independently selected from the group consisting of a hydrogen atom and a group of formula $-R^2NY_2$, wherein each Y is independently a hydrogen atom or Y', and each Y' is a group of formula

$-CH_2CH(OH)R^2-OH$

wherein $R^2$ is independently a divalent hydrocarbon group having 1 to 10 carbon atoms, and the proviso that every Y is not H.

**12.** Hair treatment composition according to claim 11, wherein Y' is a group of a formula $-CH_2CH(OH)\ CH_2OH$, and the functionalised silicone is of the pendant type, wherein n is from 200 to 500, p is from 20 to 50 and q, r and s are equal to zero.

**13.** Hair treatment composition according to any one of the preceding claims additionally comprising a hair bleaching component and/or a hair dyeing component.

**14.** Hair treatment kit comprising:

(a) an oxidative bleaching composition
(b) a dye composition; and

a hair treatment composition according to any one of the preceding claims comprised within component (a) and/or

within component (b) and/or provided as a separate component.

## Patentansprüche

1. Haarbehandlungszusammensetzung in Form einer Öl-in-Wasser-Emulsion, umfassend ein funktionalisiertes Silikonpolymer mit einer Grenzflächenspannung (GFS) von 1 bis 12 mN/m und einer Viskosität von 400 bis 150.000 mPa.s bei 30 °C, wobei sich das funktionalisierte Silikonpolymer dauerhaft auf Haar ablagert, und wobei das Silikonpolymer eine Teilchengröße von mehr als 2 $\mu$m besitzt, wobei die Haarbehandlungszusammensetzung, basierend auf dem Gewicht der wässrigen Dispersionsphase, zusätzlich zu 0,1 bis 15 % einen Emulgator umfasst, wobei der Emulgator ein Tensidsystem umfasst, das eines oder mehrere von einem anionischen Tensid, kationischen Tensid, einem amphoteren Tensid, einem wasserlöslichen polymeren Tensid, einem wasserlöslichen silikonhaltigen Tensid und einem nichtionischen Tensid beinhaltet, wobei das Tensidsystem ein Amidoamin nach der Formel $R_1CONH(CH_2)mN(R_2)_2$ umfasst, worin $R_1$ ein Rest von $C_8$- bis $C_{24}$-Fettsäuren ist, $R_2$ $C_1$- bis $C_4$-Alkyl ist und m eine ganze Zahl von 1 bis 4 ist und wobei das Tensidsystem in der Lage ist, eine Flüssigkristallstruktur um die Silikontröpfchen herum zu bilden.

2. Haarbehandlungszusammensetzung nach Anspruch 1, wobei die GFS im Bereich von 1 bis 10 mN/m, vorzugsweise im Bereich von 1 bis 8 mN/m, am meisten bevorzugt im Bereich von 1 bis 4 mN/m liegt.

3. Haarbehandlungszusammensetzung nach einem der vorstehenden Ansprüche, wobei das funktionalisierte Silikon eine Viskosität im Bereich von 4000 bis 25.000 mPa.s bei 30 °C aufweist.

4. Haarbehandlungszusammensetzung nach einem der vorstehenden Ansprüche, wobei das funktionalisierte Silikon in einer Menge im Bereich von 0,1 bis 20 Gew.-%, vorzugsweise von 0,5 bis 7,5 Gew.-% vorhanden ist.

5. Haarbehandlungszusammensetzung nach Anspruch 1, wobei das Tensidsystem keine quartären Ammoniumverbindungen der folgenden Formel umfasst:

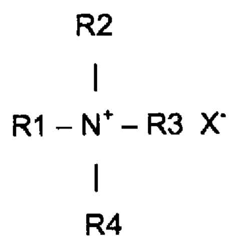

worin R1 eine Alkyl- oder Alkenylgruppe mit etwa 8 bis 22 Kohlenstoffatomen ist, R2-R4 jeweils unabhängig eine Alkyl- oder Hydroxyalkylgruppe mit etwa 1 bis 4 Kohlenstoffatomen sind und $X^-$ ein salzbildendes Anion ist.

6. Haarbehandlungszusammensetzung nach Anspruch 1 oder 5, wobei das Tensidsystem quartäre Ammoniumverbindungen der folgenden Formel umfasst:

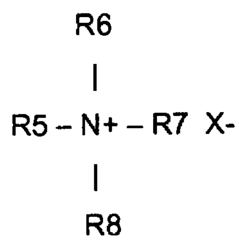

worin R5, R6 jeweils unabhängig eine Alkyl- oder Alkenylgruppe mit etwa 8 bis 22 Kohlenstoffatomen sind, R7 eine Alkyl- oder Alkenylgruppe mit etwa 8 bis 22 Kohlenstoffatomen oder eine Alkyl- oder Hydroxyalkylgruppe mit etwa 1 bis 4 Kohlenstoffatomen ist, R8 eine Alkyl- oder Hydroxyalkylgruppe mit etwa 1 bis 4 Kohlenstoffatomen ist und X- ein salzbildendes Anion ist.

7. Haarbehandlungszusammensetzung nach einem der vorstehenden Ansprüche, wobei das funktionalisierte Silikon ein organomodifiziertes Silikon vom seitenständigen oder Pfropftyp nach der folgenden Formel ist:

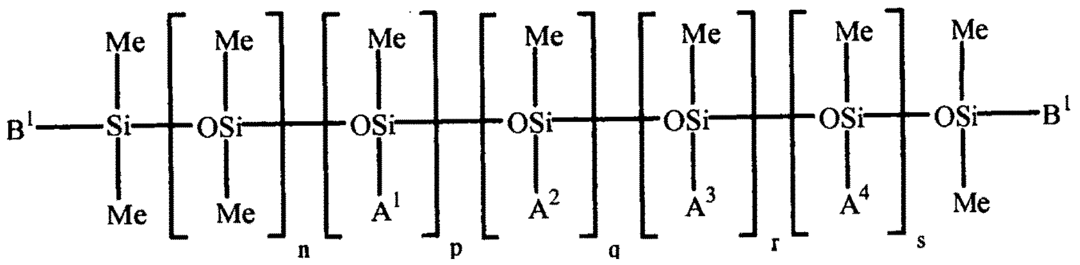

oder ein Blockcopolymer-Typ nach der folgenden Formel:

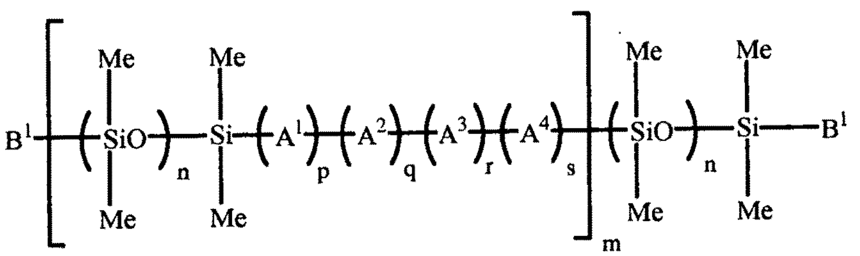

worin m größer als oder gleich 1 ist, n etwa 50 bis 2000 ist, p etwa 0 bis 50 ist, q etwa 0 bis 50 ist, r etwa 0 bis 50 ist, s etwa 0 bis 50 ist, wobei p + q + r + s größer oder gleich 1 ist, $B^1$ für H, OH, eine Alkyl- oder eine Alkoxygruppe steht; $A^1$, $A^2$, $A^3$ und $A^4$ eine gerade, verzweigte oder mono- oder polycyclische aliphatische, einfach oder mehrfach ungesättigte Alkyl-, Aryl-, Heteroalkyl-, heteroaliphatische oder heteroolefinische Einheit sein können, die 3 bis 150 Kohlenstoffatome zusammen mit 0 bis 50 Heteroatomen umfasst, einschließlich eines oder mehrerer polarer Substituenten, ausgewählt aus elektronenziehenden, elektronenneutralen oder elektronenspendenden Gruppen mit Hammett-Sigma-Para-Werten zwischen -1,0 und +1,5.

8. Haarbehandlungszusammensetzung nach Anspruch 7, wobei die polaren Substituenten die Gruppen $\alpha^1$, $\alpha^2$, $\alpha^3$ und $\alpha^4$; S-verknüpfte Gruppen, einschließlich $S\alpha^1$, SCN, $SO_2\alpha^1$, $SO_3\alpha^1$, $SS\alpha 1^1$, $SO\alpha^1$, $SO_2N\alpha^1\alpha^2$, $SN\alpha^1\alpha^2$, $S(N\alpha^1)\alpha^2$, $S(O)(N\alpha^1)\alpha^2$, $S\alpha^1(N\alpha^2)$, $SON\alpha^1\alpha^2$; O-verknüpfte Gruppen, einschließlich $O\alpha^1$, $OO\alpha^1$, OCN, $ON\alpha^1\alpha^2$; N-verknüpfte Gruppen, einschließlich $N\alpha^1\alpha^2$, $N\alpha^1\alpha^2\alpha^3+$, NC, $N\alpha^1O\alpha^2$, $N\alpha^1S\alpha^2$, NCO, NCS, $NO_2$, $N{=}N\alpha^1$, $N{=}NO\alpha^1$, $N\alpha^1CN$, $N{=}C{=}N\alpha^1$, $N\alpha^1N\alpha^2\alpha^3$, $N\alpha^1N\alpha^2N\alpha^3\alpha^4$, $N\alpha^1N{=}N\alpha^2$; COX, $CON_3$, $CON\alpha^1\alpha^2$, $CON\alpha^1CO\alpha^2$, $C({=}N\alpha^1)N\alpha^1\alpha^2$, CHO, CHS, CN, NC und X umfassen, wobei:

$\alpha^1$, $\alpha^2$, $\alpha^3$ und $\alpha^4$ eine gerade, verzweigte oder mono- oder polycyclische aliphatische, einfach oder mehrfach ungesättigte Alkyl-, Aryl-, Heteroalkyl-, heteroaliphatische oder heteroolefinische Einheit sein können, die 3 bis 150 Kohlenstoffatome zusammen mit 0 bis 50 Heteroatomen, insbesondere O, N, S, P, umfasst, und
X für F, Cl, Br oder I steht, wobei
H für Wasserstoff steht, O für Sauerstoff steht, N für Stickstoff steht, C für Kohlenstoff steht, S für Schwefel steht, Cl für Chlor steht, Br für Brom steht, I für Iod steht, F für Fluor steht.

9. Haarbehandlungszusammensetzung nach Anspruch 8, wobei die polaren Substituenten ausgewählt sind aus Polyoxyalkylen, primärem und sekundärem Amin, Amid, quatärnärem Ammonium, Carboxyl, Sulfonat, Sulfat, Kohlenhydrat, Phosphat und Hydroxyl und Mischungen davon.

10. Haarbehandlungszusammensetzung nach Anspruch 9, wobei die polaren Substituenten Aminsubstituenten umfassen.

11. Haarbehandlungszusammensetzung nach Anspruch 8, wobei die polaren Substituenten Amino-, Polyolsubstituenten der folgenden Formel umfassen:

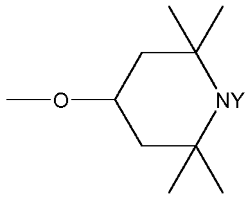

oder

- $NYR^1$

worin jedes $R^1$ unabhängig ausgewählt ist aus der Gruppe bestehend aus einem Wasserstoffatom und einer Gruppe der Formel $-R^2NY_2$, worin jedes Y unabhängig ein Wasserstoffatom oder Y' ist und jedes Y' eine Gruppe der Formel $-CH_2CH(OH)R^2-OH$ ist,
worin $R^2$ unabhängig eine zweiwertige Kohlenwasserstoffgruppe mit 1 bis 10 Kohlenstoffatomen ist, und mit der Maßgabe, dass jedes Y nicht H ist.

**12.** Haarbehandlungszusammensetzung nach Anspruch 11, wobei Y' eine Gruppe der Formel $-CH_2CH(OH)\ CH_2OH$ ist und das funktionalisierte Silikon vom seitenständigen Typ ist, wobei n von 200 bis 500 ist, p von 20 bis 50 ist und q, r und s gleich null sind.

**13.** Haarbehandlungszusammensetzung nach einem der vorstehenden Ansprüche, zusätzlich umfassend einen Haarbleichbestandteil und/oder einen Haarfärbebestandteil.

**14.** Haarbehandlungsset, umfassend:

(a) eine oxidative Bleichmittelzusammensetzung
(b) eine Farbstoffzusammensetzung; und

eine Haarbehandlungszusammensetzung nach einem der vorstehenden Ansprüche, die in Bestandteil (a) und/oder in Bestandteil (b) enthalten ist und/oder als ein separater Bestandteil bereitgestellt ist.

## Revendications

**1.** Composition de traitement capillaire sous la forme d'une émulsion d'huile dans l'eau comprenant un polymère de silicone fonctionnalisée possédant une tension interfaciale (IFT) de 1 à 12 mN/m et une viscosité de 400 à 150 000 mPa.s à 30 °C, dans laquelle le polymère de silicone fonctionnalisée se dépose durablement sur les cheveux, et dans laquelle ledit polymère de silicone a une taille de particules supérieure à 2 μm, où la composition de traitement capillaire comprend en outre 0,1 à 15 % sur la base du poids de la phase continue aqueuse de l'émulsifiant, dans laquelle l'émulsifiant comprend un système tensioactif incluant un ou plusieurs parmi un agent tensioactif anionique, un agent tensioactif cationique, un agent tensioactif amphotère, un agent tensioactif polymère hydrosoluble, un agent tensioactif hydrosoluble contenant de la silicone et un agent tensioactif non ionique, dans laquelle le système tensioactif comprend une amido-amine selon la formule $R_1CONH(CH_2)_mN(R_2)_2$ dans laquelle $R_1$ est un résidu d'acides gras en $C_8$ à $C_{24}$, $R_2$ est un alkyle en $C_1$ à $C_4$ et m est un nombre entier allant de 1 à 4 et dans laquelle le système tensioactif est susceptible de former une structure de cristal liquide autour des gouttelettes de silicone.

**2.** Composition de traitement capillaire selon la revendication 1, dans laquelle la tension interfaciale est dans la gamme de 1 à 10 mN/m, de préférence dans la gamme de 1 à 8 mN/m, le plus préférablement dans la gamme de 1 à 4 mN/m.

**3.** Composition de traitement capillaire selon l'une quelconque des revendications précédentes, dans laquelle la silicone fonctionnalisée a une viscosité dans la gamme de 4000 à 25 000 mPa.s, à 30 °C.

**4.** Composition de traitement capillaire selon l'une quelconque des revendications précédentes, dans laquelle la silicone fonctionnalisée est présente en une quantité allant de 0,1 à 20 % en poids, de préférence de 0,5 à 7,5 % en poids.

**5.** Composition de traitement capillaire selon la revendication 1, dans laquelle le système tensioactif ne comprend pas

de composés d'ammonium quaternaire de formule :

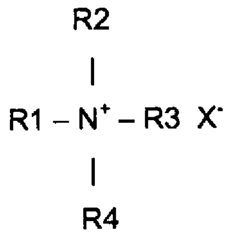

où R1 est un groupe alkyle ou alcényle ayant d'environ 8 à 22 atomes de carbone, R2-R4 sont chacun indépendamment un groupe hydroxyalkyle ou alkyle ayant d'environ 1 à 4 atomes de carbone, et $X^-$ est un anion salifiable.

**6.** Composition de traitement capillaire selon la revendication 1 ou 5, dans laquelle le système tensioactif comprend des composés d'ammonium quaternaire de formule

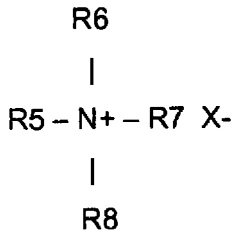

où R5, R6 sont chacun indépendamment un groupe alkyle ou alcényle ayant d'environ 8 à 22 atomes de carbone, R7 est un groupe alkyle ou alcényle ayant d'environ 8 à 22 atomes de carbone ou un groupe hydroxyalkyle ou alkyle ayant d'environ 1 à 4 atomes de carbone, R8 est un groupe hydroxyalkyle ou alkyle ayant d'environ 1 à 4 atomes de carbone, et X- est un anion salifiable.

**7.** Composition de traitement capillaire selon l'une quelconque des revendications précédentes, dans laquelle la silicone fonctionnalisée est une silicone organomodifiée du type greffé ou greffe selon la formule suivante :

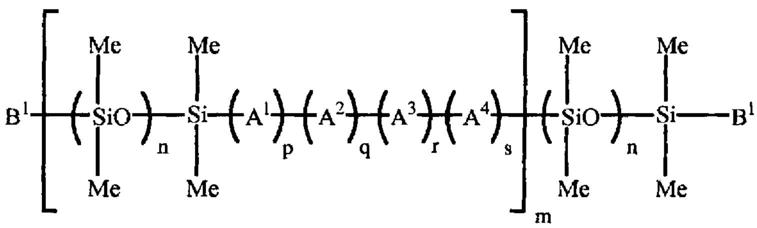

ou un type de copolymère séquencé selon la formule suivante :

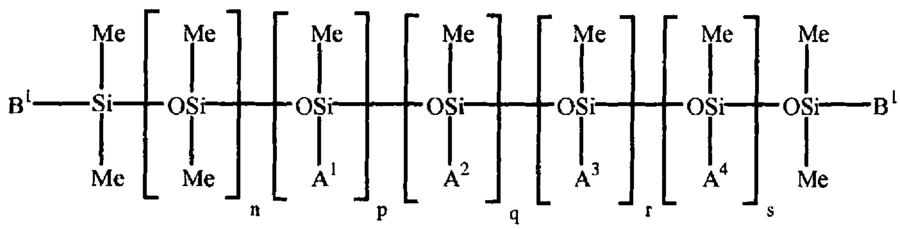

où m est supérieur ou égal à 1, n est environ 50 à 2000, p est environ 0 à 50, q est environ 0 à 50, r est environ 0 à 50, s est environ 0 à 50, où p + q + r + s est supérieur ou égal à 1, B1 est H, OH, un groupe alkyle ou alcoxy ; $A^1$, $A^2$, $A^3$ et $A^4$ sont un fragment alkyle, aryle, hétéroalkyle, hétéroaliphatique ou hétéro-oléfinique linéaire, ramifié ou mono ou polycyclique aliphatique, mono ou polyinsaturé comprenant 3 à 150 atomes de carbone conjointement

avec 0 à 50 hétéroatomes y compris un ou plusieurs substituants polaires choisis parmi les groupes capteurs d'électron, électroniquement neutres, ou donneur d'électron avec des para valeurs sigma de Hammett comprises entre -1,0 et +1,5.

**8.** Composition de traitement capillaire selon la revendication 7, dans laquelle les substituants polaires comprennent des groupes $\alpha^1$, $\alpha^2$, $\alpha^3$ et $\alpha^4$ ; des groupes liés à S incluant $S\alpha^1$, SCN, $SO_2\alpha^1$, $SO_3\alpha^1$, $SS\alpha 1^1$, $SO\alpha^1$, $SO_2N\alpha^1\alpha^2$, $SN\alpha^1\alpha^2$, $S(N\alpha^1)\ \alpha^2$, $S(O)(N\alpha^1)\ \alpha^2$, $S\alpha^1(N\alpha^2)$, $SON\alpha^1\alpha^2$ ; des groupes liés à O incluant $O\alpha^1$, $OO\alpha^1$, OCN, $ON\alpha^1\alpha^2$ ; des groupes liés à N incluant $N\alpha^1\alpha^2$, $N\alpha^1,\alpha^2,\alpha^3+$, NC, $N\alpha^1O\alpha^2$, $N\alpha^1S\alpha^2$, NCO, NCS, $NO_2$, $N{=}N\alpha^1$, $N{=}NO\alpha^1$, $N\alpha^1CN$, $N{=}C{=}N\alpha^1$, $N\alpha^1N\alpha^2\alpha^3$, $N\alpha^1N\alpha^2N\alpha^3\alpha^4$, $N\alpha^1N{=}N\alpha^2$ ; COX, $CON_3$, $CON\alpha^1\alpha^2$, $CON\alpha^1CO\alpha^2$, $C({=}N\alpha^1)N\alpha^1\alpha^2$, CHO, CHS, CN, NC, et X, où :

$\alpha^1$, $\alpha^2$, $\alpha^2$ et $\alpha^4$ peuvent être un fragment alkyle, aryle, hétéroalkyle, hétero-aliphatique ou hétero-oléfinique aliphatique, mono ou polyinsaturé, linéaire, ramifié ou mono ou polycyclique comprenant 3 à 150 atomes de carbone conjointement avec 0 à 50 hétéroatomes, spécialement O, N, S, P, et
X est F, Cl, Br ou I, où
H est l'hydrogène, O est l'oxygène, N est l'azote, C est le carbone, S est le soufre, Cl est le chlore, Br est le brome, I est l'iode, F est le fluor.

**9.** Composition de traitement capillaire selon la revendication 8, dans laquelle les substituants polaires sont choisis parmi un polyoxyalkylène, une amine primaire et secondaire, un amide, un ammonium quaternaire, un carboxyle, un sulfonate, un sulfate, un hydrate de carbone, un phosphate, et un hydroxyle et des mélanges de ceux-ci.

**10.** Composition de traitement capillaire selon la revendication 9, dans laquelle les substituants polaires comprennent des substituants amine.

**11.** Composition de traitement capillaire selon la revendication 8, dans laquelle les substituants polaires comprennent des substituants amino-, polyol- de formule :

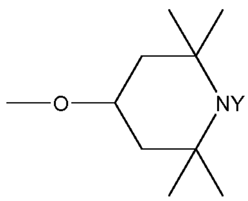

ou

$$-NYR^1$$

dans laquelle chaque $R^1$ est indépendamment choisi parmi le groupe constitué d'un atome d'hydrogène et d'un groupe de formule $-R^2NY_2$, dans laquelle chaque Y est indépendamment un atome d'hydrogène ou Y', et chaque Y' est un groupe de formule $-CH_2CH(OH)R^2-OH$
dans laquelle $R^2$ est indépendamment un groupe hydrocarboné divalent comportant 1 à 10 atomes de carbone, et à la condition que chaque Y ne soit pas H.

**12.** Composition de traitement capillaire selon la revendication 11, dans laquelle Y' est un groupe de formule $-CH_2CH(OH)CH_2OH$, et la silicone fonctionnalisée est du type greffé, dans laquelle n va de 200 à 500, p va de 20 à 50 et q, r et s sont égaux à zéro.

**13.** Composition de traitement capillaire selon l'une quelconque des revendications précédentes comprenant en outre un composant d'éclaircissement des cheveux et/ou un composant de teinture capillaire.

**14.** Trousse de traitement capillaire comprenant :

(a) une composition d'éclaircissement oxydante
(b) une composition de teinture ; et

une composition de traitement capillaire selon l’une quelconque des revendications précédentes comprise au sein du composant (a) et/ou au sein du composant (b) et/ou fournie en tant que composant indépendant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0275707 A
- WO 9949836 A
- WO 9827945 A


### Non-patent literature cited in the description


- HLB-Wert. Römpp Chemie Lexikon. Thieme Verlag, 1995
- Hammett Gleichung. Römpp Chemie Lexikon. Georg Thieme Verlag, 1995
- **SAGARIN.** Cosmetic Science and Technology. Interscience, vol. 2, 308-310
- Chemical and Physical Behaviour of Human Hair. 250-259
- The Chemistry and Manufacture of Cosmetics. vol. IV, 841-920
- cosmetics: Science and Technology. vol. II, 279-343
- The Science of Hair Care. 235-261
- Hair Dyes. Noyes Data Corp, 1973, 3-91, 113-139
- **C. E. MORTIMER.** Chemistry. 277
- **F. K. HANSEN ; G. RODSRUN.** Surface tension by pendant drop. A fast standard instrument using computer image analysis. *Journal of Colloid and Interface Science,* January 1991, vol. 141 (1), 1-9